# EUROPÄISCHE PATENTANMELDUNG

(11) **EP 1 312 680 A1**
(43) Veröffentlichungstag der Anmeldung: **21.05.2003**
(21) Anmeldenummer: 01127237.4
(22) Anmeldetag: 16.11.2001
(51) Int. Cl.: C12P 21/02, C07K 1/02, C07K 14/00

(54) **Verfahren zur Synthese von all-D-Peptiden und Peptidnukleinsäuren**

(71) Anmelder: Noxxon Pharma AG, 13355 Berlin (DE)
(72) Erfinder: Klussmann, Sven, Dr., 10709 Berlin (DE); Bordusa, Frank, Dr., 06242 Rossbach (DE); Burmeister, Jens, Dr., 50161 Köln (DE)
(74) Vertreter: Bohmann, Armin K., Dr.

(57) **Zusammenfassung**

Die vorliegende Erfindung betrifft ein Verfahren zur Synthese eines all-D-Polypeptids unter Verwendung eines Enzyms umfassend die Schritte:
(a) Bereitstellen einer Aminokomponente, wobei die Aminokomponente aus mindestens einer D-Aminosäure, bevorzugter Weise einem all-D-Polypeptid besteht,
(b) Bereitstellen der Carboxykomponente, wobei die Carboxykomponente eine Abgangsgruppe umfasst und aus mindestens einer D-Aminosäure, bevorzugter Weise einem all-D-Polypeptid besteht, und
(c) Umsetzen der Aminokomponente und der Carboxykomponente mit dem Enzym unter Ausbildung einer Peptidbindung zwischen der Aminokomponente und der Carboxykomponente und unter Abspaltung der Abgangsgruppe von der Carboxykomponente, wobei das all-D-Polypeptid erhalten wird.

## Beschreibung

Die Erfindung betrifft Verfahren zur Synthese eines all-D-Polypeptids unter Verwendung eines Enzyms, die Verwendung von Enzymen zur Synthese von all-D-Polypeptiden sowie die Verwendung von Abgangsgruppen zur Synthese von all-D-Polypeptiden.

Die Synthese von Peptiden und Proteinen besitzt zunehmende Bedeutung für das systematische Studium von Struktur-Funktionsbeziehungen der Polypeptide als funktionelle Genprodukte und trägt entscheidend zur Entdeckung neuer wirksamer Therapeutika bei (vgl. H.-D. Jakubke, *Peptide: Chemie und Biologie,* Spektrum Akademischer Verlag, Heidelberg, Berlin, Oxford, 1996). Ein wesentliches Problem bei der therapeutischen Anwendung dieser Biopolymere ist jedoch deren inhärente Instabilität in biologischen Medien. Die Spiegelung der Konfiguration (Austausch der codierten L-Aminoäuren gegen analoge D-Aminosäuren) führt zu einer deutlichen Stabilitätserhöhung der resultierenden funktionalen spiegelbildlichen Proteine gegenüber proteolytischem Abbau und einhergehender Inaktivierung.

Neben der Verwendung von D-Polypeptiden als Therapeutika werden diese als molekularbiologische Werkzeuge zunehmend bedeutsamer. Eine Anwendung der D-Polypeptide, hierin auch als all-D-Polypeptide bezeichnet, ist die Herstellung von sogenannten Spiegelmeren. Dabei handelt es sich um einzelsträngige Nukleinsäuren, die hochaffin und hochspezifisch an das Zielmolekül binden. Dabei wird so vorgegangen, dass das jeweilige Zielmolekül, gegen das ein Spiegelmer erzeugt werden soll, in seiner all-D-Form synthetisiert wird, sodann eine D-Nukleinsäure-Bibliothek, die typischerweise etwa 10¹⁵ verschiedene Sequenzen enthält, verwendet wird, um aus dieser Bibliothek an das all-D-Polypeptid bindende D-Nukleinsäuren zu identifizieren und zu isolieren. Die bindende(n) D-Nukleinsäure(n) wird/werden sodann in ihre L-Enantiomere überführt und binden infolge der chiralen Reziprozität an das Zielmolekül, in dem Falle, dass es sich bei dem Zielmolekül um ein Protein handelt, an das entsprechende all-L-Protein. (S. Klußmann et al., *Nat. Biotechnol*. **1996**, 14, 1112; A. Nolte et al., *Nat. Biotechnol.* **1996,** 14, 1116).

Grundsätzlich lassen sich die für die Synthese von all-L-Polypeptiden entwickelten chemischen Methoden, aufgrund ihrer fehlenden Stereoselektivität, auch auf die Darstellung ihrer all-D-Analoga übertragen. Aufgrund vergleichbarer Reaktivitäten der zur Synthese eingesetzten vollgeschützten D-Aminosäurebausteine resultieren daher zugleich ähnliche Limitationen wie für die Synthese von all-L-Polypeptiden. Während Peptide mit einer durchschnittlichen Kettenlänge von 50-60 Aminosäuren direkt durch Festphasenpeptidsynthese zugänglich sind, führt eine weitere Kettenverlängerung wegen der nicht in jedem Fall quantitativen Kupplungsausbeuten häufig zur Akkumulation einer Vielzahl von Nebenprodukten, die sowohl zu einer Verringerung der Syntheseausbeuten führen wie auch die Reinigung des gewünschten Produktes erschweren bzw. verhindern. Gegenwärtige Methoden beruhen daher auf der Kondensation von synthetisch bereitgestellten Peptidfragmenten, wobei jedoch die Verknüpfung vollgeschützter Peptidfragmente wegen der häufig sehr geringen Löslichkeit der Edukte nur in Ausnahmefällen möglich ist.

Die basierend auf dem erstmals 1953 vorgeschlagenen Konzept der Molekülklammer für chemische CN-Ligationen von ungeschützten Peptidfragmenten (T. Wieland et al., *Annalen* **1953,** 583, 129; M. Brenner et al., *Helv. Chim. Acta* **1957,** 40, 1497) entwickelten Methoden des Amin- und Thioleinfangs (D.S. Kemp et al., *J. Org. Chem.* **1975,** 40, 3465; N. Fotouhi et al., *J. Org. Chem.* **1989,** 54, 2803), der natürlichen chemischen Ligation (M. Schnölzer, S.B.H. Kent, *Science* **1992,** 256,221; P.E. Dawson et al., *Science* **1994,** 266, 776) oder auch der Aldehyd-Methode (C.-F. Liu, J.P. Tam, *Proc. Natl. Acad. Sci. USA* **1994,** 91, 6584) verlaufen zwar selektiv, erfordern aber für deren Realisierung ganz bestimmte N- bzw. C-terminale Aminosäurereste, so daß ihre Anwendbarkeit sequenzspezifischen Voraussetzungen unterliegt. Bei der z.Z. favorisierten nativen chemischen Ligation erfolgt die Verknüpfung eines synthetischen Peptids mit einer C-terminalen Thioestergruppierung mit einem zweiten Peptid oder Protein, das einen N-terminalen Cysteinrest enthalten muß. Für all-D-Peptide nicht anwendbar ist hingegen die auf diesem Prinzip basierende Intein-vermittelten Protein-Ligation (*expressed protein ligation*, EPL; vgl. u. a. T.W. Muir et al., *Proc. Natl. Acad. Sci. USA* **1998,** 95, 6705; G.J. Cotton et al., *J. Am. Chem. Soc.* **1999,** 121, 1100), bei der die Thioestergruppierung der Carboxykomponente aus einem rekombinanten Protein, das mit einem spaltungskompetenten Intein fusioniert ist und durch thiolytische Spaltung gebildet wird. Neben der Notwendigkeit eines Cysteinrestes am N-Terminus der Aminokomponente liegt ein weiterer genereller Nachteil in der nicht auszuschließenden partiellen Epimerisierung des C-terminalen Aminosäurerestes, da der sich bildende Thioester (zumindest bei Einsatz von Thiophenol als Katalysator) nicht nur erst nach der Umesterung, sondern auch direkt von der terminalen α-Aminogruppe der zugesetzten Aminokomponente nukleophil angegriffen werden kann.

Katalytische Verfahren bieten den Vorteil einer höheren Flexibilität hinsichtlich der zu synthetisierenden Peptidbindung, obgleich aus der Natur zumindest bislang keine universelle Peptidligase mit präparativer Relevanz bekannt ist. So zeigen katalytische Antikörper (vgl. u.a. P.G. Schultz, R.A. Lerner, *Science* **1995,** 269, 1835; G. MacBeath, D. Hilvert, *Chem. Biol*. **1996**, 3, 433; D.B. Smithrub et al., *J. Am. Chem. Soc.* **1997**, 119, 278) CN-Ligaseaktivität, ebenso wie synthetische Peptidligasen basierend auf einem *coiled-coil*-Motif von GCN4 (K. Severin et al., *Nature* **1997**, 389, 706) bzw. auf einer Peptidmatrize bestehend aus einem stark sauren *coiled-coil-Peptid (S.* Yao, J. Chmielewski, *Biopolymers* **1999,** *51,* 370). Bei allen diesen Fällen handelt es sich zweifelsohne um interessante Ansatzpunkte für das Design von Peptidligasen, die für Ligationen allerdings spezielle Voraussetzungen erfordern und deren allgemeine Anwendbarkeit folglich sehr stark limitiert ist. Die Nutzung des reversen Katalysepotentials von Peptidasen (vgl. u.a. W. Kullmann, *Enzymatic Peptide Synthesis,* CRC Press, Boca Raton, **1987;** H.-D. Jakubke, *Enzymatic Peptide Synthesis,* in: *The Peptides: Analysis, Synthesis, Biology*, Vol. 9, (Eds.: S. Udenfriend, J. Meienhofer), Academic Press, New York, **1987,** Chapter 3) bietet zwar die prinzipielle Möglichkeit, Peptidsegmente unter speziellen Voraussetzungen enzymatisch zu verknüpfen, doch beschränken sich die beschriebenen Beispiele wie bei den zuvor genannten biokatalytischen Verfahren auf die Kondensation von all-L-Peptiden bzw. die Knüpfung von entweder D-konfigurierten Aminokomponenten oder D-Carboxykomponenten, wobei in keinem Falle eine all-D-Aminokomponente mit einer all-D-Carboxykomponente verknüpft wurde. Im übrigen lag die Carboxykomponente immer nur als einzelner Aminosäurebaustein, d. h. als D-Aminosäure vor und nicht als all-D-Peptid. (vgl. u.a. Bordusa et al., *Biol. Chem.* **1997,** 378, 1193; F. Bordusa, H.-D. Jakubke, *Bioorg. Med. Chem.* **1998,** 6, 1775; M. Thormann et al., *Biochemistry* **1999,** 38, 6056; R. Günther et al., *Eur. J. Biochem.* **2000,** 267, 3496).

Der vorliegenden Erfindung liegt die Aufgabe zugrunde, ein Verfahren zur enzymatischen Synthese von all-D-Polypeptiden bereitzustellen, welches die Nachteile der im Stand der Technik beschriebenen Verfahren überwindet. Weiterhin ist es Aufgabe der vorliegenden Erfindung, ein Verfahren bereitzustellen, bei dem eine sequenzunabhängige Synthese, insbesondere Ligation, eines all-D-Polypeptids erfolgt.

Die Aufgabe wird erfindungsgemäß in einem ersten Aspekt gelöst durch ein Verfahren zur Synthese eines all-D-Polypeptids unter Verwendung eines Enzyms umfassend die Schritte
a) Bereitstellen einer Aminokomponente, wobei die Aminokomponente aus mindestens einer D-Aminosäure, bevorzugter Weise einem all-D-Polypeptid besteht,
b) Bereitstellen der Carboxykomponente, wobei die Carboxykomponente eine Abgangsgruppe umfasst und die Carboxykomponente aus mindestens einer D-Aminosäure, bevorzugter Weise einem all-D-Polypeptid, besteht, und
c) Umsetzen der Aminokomponente und der Carboxykomponente mit dem Enzym unter Ausbildung einer Peptidbindung zwischen der Aminokomponente und der Carboxykomponente unter Abspaltung der Abgangsgruppe von der Carboxykomponente, wobei das all-D-Polypeptid erhalten wird.

In einer Ausführungsform ist vorgesehen, dass das erfindungsgemäße Verfahren weiter den Schritt umfasst:
(a) Isolieren des all-D-Polypeptids.

In einer weiteren Ausführungsform ist vorgesehen, dass das Enzym ausgewählt ist aus der Gruppe, die Proteasen, Peptidasen und Hydrolasen umfasst.

In einer noch weiteren Ausführungsform ist vorgesehen, dass das Enzym ausgewählt ist aus der Gruppe, die Cysteinproteasen und Serinproteasen umfasst.

Schließlich ist in einer Ausführugnsform vorgesehen, dass das Enzym Clostripain, insbesondere Clostripain aus *Clostridium histolyticum* ist.

In einer bevorzugten Ausführungsform ist vorgesehen, dass das Enzym eine Prolylendopeptidase, insbesondere eine Prolylendopeptidase aus *Flavobacterium meningoseptum* ist.

In einer Ausführungsform ist vorgesehen, dass die Abgangsgruppe ausgewählt ist aus der Gruppe, die 4-Guanidinophenylester , 4-Amidinophenylester, 4-Guanidinophenylthioester und 4-Amidinophenylthioester und Strukturhomologe davon umfasst.

In einer weiteren Ausführungsform ist vorgesehen, dass die die Abgangsgruppe enthaltende Carboxykomponente die folgende Struktur aufweist:

Y-(D-Xaa)ₙ-R

wobei Y = eine N-terminale Schutzgruppe, insbesondere Benzoyl ist,
Xaa = eine beliebige α-Aminosäure, β-Aminosäure oder ein Derivat derselben ist
R = eine Abgangsgruppe ist, insbesondere eine Abgangsgruppe ist, die ausgewählt ist aus der Gruppe, die 4-Guanidinophenylester, 4-Amidinophenylester, 4-Guanidinophenylthioester, 4-Amidinophenylthioester und Strukturhomologe davon umfasst
n = eine ganze Zahl, bevorzugter Weise von 1 bis 100.

Schließlich ist in einer Ausführungsform vorgesehen, dass die N^{α}-Aminofunktion der Aminokomponente ungeschützt vorliegt, wobei N^{α}-Aminofunktion mit der Carboxyfunktion der Carboxykomponente reagiert.

Weiterhin wird die Aufgabe in einem zweiten Aspekt gelöst durch ein Verfahren zur Synthese eines all-D-Polypeptids unter Verwendung eines Enzyms umfassend die Schritte
a) Bereitstellen einer Aminokomponente, wobei die Aminokomponente aus mindestens einer D-Aminosäure, bevorzugter Weise einem all-D-Polypeptid besteht,
(b) Bereitstellen der Carboxykomponente, wobei die Carboxykomponente eine Abgangsgruppe umfasst und die Carboxykomponente aus mindestens einer D-Aminosäure, bevorzugter Weise aus einem all-D-Polypeptid besteht, und
c) Umsetzen der Aminokomponente und der Carboxykomponente mit dem Enzym unter Ausbildung einer Peptidbindung zwischen der Aminokomponente und der Carboxykomponente und Abspalten der Abgangsgruppe von der Carboxykomponente, wobei das all-D-Polypeptid erhalten wird,
wobei die Aminokomponente nach einem Verfahren gemäß dem ersten Aspekt der vorliegenden Erfindung hergestellt wird oder ist.

Weiterhin wird die Aufgabe in einem dritten Aspekt gelöst durch ein Verfahren zur Synthese eines all-D-Polypeptids unter Verwendung eines Enzyms, wobei es sich bevorzugter Weise um ein Verfahren gemäß dem zweiten Aspekt der vorliegenden Erfindung handelt, umfassend die Schritte
a) Bereitstellen einer Aminokomponente, wobei die Aminokomponente aus mindestens einer D-Aminosäure, bevorzugter Weise einem all-D-Polypeptid besteht,
(b) Bereitstellen der Carboxykomponente, wobei die Carboxykomponente eine Abgangsgruppe umfasst und die Carboxykomponente aus mindestens einer D-Aminosäure, bevorzugter Weise aus einem all-D-Polypeptid besteht, und
c) Umsetzen der Aminokomponente und der Carboxykomponente mit dem Enzym unter Ausbildung einer Peptidbindung zwischen der Aminokomponente und der Carboxykomponente und Abspalten der Abgangsgruppe von der Carboxykomponente, wobei das all-D-Polypeptid erhalten wird,
wobei die Carboxykomponete nach einem Verfahren gemäß dem ersten Aspekt der vorliegenden Erfindung hergestellt wird oder ist.

In einem vierten Aspekt wird die Aufgabe erfindungsgemäß gelöst durch die Verwendung eines Enzyms zur Synthese eines all-D-Polypeptids.

In einer Ausführungsform ist vorgesehen, dass das all-D-Polypeptid durch Ligation einer Aminokomponente und einer Carboxykomponente hergestellt wird und die Carboxykomponente eine Abgangsgruppe umfasst.

In einer weiteren Ausführungsform ist vorgesehen, dass die Abgangsgruppe an einem PNA-Strang vorhanden ist und der die Abgangsgruppe tragende PNA-Strang als Carboxykomponente mit einem PNA-Monomer oder mit einem zweiten PNA-Strang als Aminokomponente unter Verwendung eines Enzyms umgesetzt wird.

In einer noch weiteren Ausführungsform ist vorgesehen, dass die Abgangsgruppe ausgewählt ist aus der Gruppe, die 4- Guanidinophenylester, 4-Amidinophenylester, 4-Guanidinophenylthioester und 4-Amidinophenylthioester und Strukturhomologe davon umfasst.

In einem fünften Aspekt der Erfindung wird die Aufgabe gelöst durch die Verwendung einer Abgangsgruppe, die ausgewählt ist aus der Gruppe, die 4-Guanidinophenylester, 4-Amidinophenylester, 4-Guanidinophenylthioester und 4-Amidinophenylthioester und Strukturhomologe davon umfasst, zur Synthese eines all-D-Polypeptids unter Verwendung eines Enzyms.

In einer Ausführungsform ist vorgesehen, dass die Abgangsgruppe an einem all-D-Polypeptid vorhanden ist und das die Abgangsgruppe tragende all-D-Polypeptid als Carboxykomponente mit einer D-Aminosäure oder mit einem all-D-Polypeptid als Aminokomponente unter Verwendung eines Enzyms umgesetzt wird.

In Ausführungsformen des vierten und fünften Aspekts der vorliegenden Erfindung ist vorgesehen, dass das Enzym Clostripain, insbesondere Clostripain aus *Clostridium histolyticum* ist.

In weiteren Ausführungsformen des vierten und fünften Aspekts der vorliegenden Erfindung ist vorgesehen dass das Enzym Prolylendopeptidase, insbesondere Prolylendopeptidase aus *Flavobacterium meningoseptum* ist.

In einem sechsten Aspekt betrifft die Erfindung ein Verfahren zur Synthese von PNA unter Verwendung eines Enzyms umfassend die Schritte:
(a) Bereitstellen einer Aminokomponente, wobei die Aminokomponente aus mindestens einem PNA-Monomer, bevorzugter Weise einem ersten PNA-Strang besteht,
(b) Bereitstellen einer Carboxykomponente, wobei die Carboxykomponente eine Abgangsgruppe umfasst und die Carboxykomponente aus mindestens einem PNA-Monomer, bevorzugter Weise einem zweiten PNA-Strang besteht, und
(c) Umsetzen der Aminokomponente und der Carboxykomponente mit dem Enzym unter Ausbildung einer Peptidbindung zwischen der Aminokomponente und der Carboxykomponente unter Abspaltung der Abgangsgruppe von der Carboxykomponente, wobei ein ligierter PNA-Strang erhalten wird.

In einer Ausführungsform ist vorgesehen, dass das Verfahren als weiteren Schritt umfasst:
(d) Isolieren des ligierten PNA-Stranges.

In einem siebten Aspekt betrifft die vorliegende Erfindung ein Verfahren zur Synthese von PNA unter Verwendung eines Enzyms umfassend die Schritte
(a) Bereitstellen einer Aminokomponente, wobei die Aminokomponente aus mindestens einem PNA-Monomer, bevorzugter Weise einem ersten PNA-Strang besteht,
(b) Bereitstellen einer Carboxykomponente, wobei die Carboxykomponente eine Abgangsgruppe umfasst und die Carboxykomponente aus mindestens einem PNA-Monomer, bevorzugter Weise aus einem zweiten PNA-Strang besteht, und
(c) Umsetzen der Aminokomponente und der Carboxykomponente mit dem Enzym unter Ausbildung einer Peptidbindung zwischen der Aminokomponente und der Carboxykomponente und Abspalten der Abgangsgruppe von der Carboxykomponente,
wobei die Aminokomponente hergestellt ist nach einem der erfindungsgemäßen Verfahren.

In einem achten Aspekt betrifft die vorliegende Erfindung ein Verfahren zur Synthese von PNA unter Verwendung eines Enzyms umfassend die Schritte
(a) Bereitstellen einer Aminokomponente, wobei die Aminokomponente aus mindestens einem PNA-Monomer, bevorzugter Weise einem ersten PNA-Strang besteht,
(b) Bereitstellen einer Carboxykomponente, wobei die Carboxykomponente eine Abgangsgruppe umfasst und die Carboxykomponente aus mindestens einem PNA-Monomer, bevorzugter Weise aus einem zweiten PNA-Strang besteht, und
(c) Umsetzen der Aminokomponente und der Carboxykomponente mit dem Enzym unter Ausbildung einer Peptidbindung zwischen der Aminokomponente und der Carboxykomponente und Abspalten der Abgangsgruppe von der Carboxykomponente,
wobei die Carboxykomponente hergestellt ist nach einem der erfindungsgemäßen Verfahren.

Ausgehend von dem bisher im Stand der Technik anerkannten Dogma, dass sowohl die Hinwie auch die Rückreaktion von Enzymen, d. h. im Falle von Proteasen und Peptidasen und allgemein Hydrolasen sowohl die Hydrolyseaktivität wie auch die Ligaseaktivität, denselben Spezifitätskriterien unterliegt, lag der vorliegenden Erfindung die überraschende Erkenntnis zugrunde, dass es möglich ist, mit einer mit einer Abgangsgruppe versehenen Carboxykomponenten, wobei die Carboxykomponente eine D-Komponente darstellt, diese Spezifitätskriterien aufzuheben. Mit anderen Worten, indem die D-Carboxykomponente mit einer Abgangsgruppe versehen wird, kann eine Ligasereaktion erfolgen, nicht jedoch die Spaltungsreaktion im Sinne einer Hydrolyse eines aus einer D-Aminokomponente und einer D-Carboxykomponente hergestellten all-D-Polypeptids. Die D-Carboxykomponente wird mit der Abgangsgruppe typischerweise dadurch versehen, daß die Abgangsgruppe ester- oder amidartig mit der C-terminalen Carboxylfunktion der Carboxykomponente verknüpft wird. Dem erfindungsgemäßen Verfahren liegt des weiteren die überraschende Erkenntnis zugrunde, dass trotz der ausgeprägten Stereospezifität von Enzymen wie Proteasen und Peptidasen für L-konfigurierte Aminosäuren und daraus aufgebauter L-Polypeptide eine Enzym-katalysierte Ligation einer sowohl D-konfigurierten Carboxy- wie auch einer D-konfigurierten Aminokomponente möglich ist, obwohl diese nach dem Verständnis der Fachleute auf dem Gebiet formal auszuschließen war (vgl. H.-D. Jakubke, *Peptide: Chemie und Biologie,* Spektrum Akademischer Verlag, Heidelberg, Berlin, Oxford, 1996).

Man hat gefunden, dass die Cysteinprotease Clostripain sowie die Serinprotease Prolylendopeptidase das vorstehend beschriebene Verhalten zeigen und somit im Rahmen der erfindungsgemäßen Verfahren zur Synthese von all-D-Polypeptiden verwendet werden können. Weitere geeignete Enzyme können im Rahmen von Screening-Prozessen unter Verwendung geeigneter Modellreaktionen, wie sie auf der Grundlage der hierin offenbarten technischen Lehre durchgeführt werden können, erhalten werden.

Im Rahmen eines derartigen Screening-Prozesses wird dabei so vorgegangen, daß die generelle Akzeptanz D-konfigurierter peptidischer Aminokomponenten durch Proteasen mittels synthetischer Modellreaktionen untersucht wird. Hierzu wird im einfachsten Fall eine aus einer Aminosäure bestehende Carboxykomponente (herkömmliche Carboxykomponenten oder Substratmimetikum) mit einer D-konfigurierten Aminokomponenten, im einfachsten Fall ein Amid einer D-Aminosäure, bevorzugter Weise aber einem all-D-Peptid, und der zu testenden Protease inkubiert. Die Akzeptanz D-konfigurierter Aminokomponenten wird durch Produktbildung im Verlauf der nachfolgenden Inkubationsphase angezeigt. Die Produktbildung selbst kann beispielsweise mittels HPLC oder anderer chromatographischer Trennmethoden analysiert werden. Alternativ kann durch Anbringen geeigneter quenchender Fluoreszenzdonor und -akzeptor-Paare an die Carboxy- bzw. Aminokomponente (z.B. 2-Aminobenzoesäure an die Carboxykomponente und 2-Nitro-Tyrosin an die Aminokomponente) durch Abnahme der Fluoreszenzintensität auf eine Produktbildung und demzufolge auf eine Akzeptanz D-konfigurierter Aminokomponenten geschlossen werden. Daneben erlauben auch ELISA-Techniken unter Verwendung produktspezifischer Antikörper wie auch massenspektroskopische Verfahren die Detektion von gebildetem Ligationsprodukt aus Carboxy- und Aminokomponente.

Aufgrund der Reversibilität der katalysierten Reaktion (zumindest bei Verwendung herkömmlicher Carboxykomponenten) kann ein Screening auch basierend auf Spaltungsexperimenten erfolgen. Das zu spaltende Peptidsubstrat besteht in solchen Fällen sowohl aus L- wie auch aus D-Aminosäuren. Die Spaltung der Peptidbindung zwischen einer L-Aminosäure und einer D-Aminosäuren kann als Akzeptanz D-konfigurierter Aminokomponenten gewertet werden. Grundsätzlich sind zur Analyse der Spaltungsreaktionen die gleichen Methoden, wenn auch z.T. in für den Fachmann bekannter modifizierter Form, wie für den o.g. synthetischen Screening-Ansatz einsetzbar.

Ohne im folgenden darauf festgelegt sein zu wollen, scheint die überraschend festgestellte Verwendungsmöglichkeit von Enzymen, insbesondere von Proteasen, Peptidasen und Hydrolasen, zur Synthese eines all-D-Polypeptids ausgehend von einer D-konfigurierten Aminokomponente sowie einer D-konfigurierten Carboxykomponente darin begründet zu liegen, dass infolge der an der D-konfigurierten Carboxykomponente vorhandenen Abgangsgruppe die native Spezifität des eingesetzten Enzyms dahingehend ausgestaltet oder geändert wird, dass das Enzym nun auch die vorstehend genannten D-konfigurierten Komponenten als Substrate akzeptiert und diese in einer katalytischen Reaktion mit dem Ergebnis ligiert, dass ein all-D-Polypeptid erhalten wird, welches eine Gesamtstruktur bestehend aus der Aminokomponente sowie der Carboxykomponente aufweist. Es ist beachtlich, dass das Enzym dabei in der Regel nicht auf die Ligation von Carboxykomponenten mit einer spezifischen Sequenz abstellt, sondern vielmehr beliebige Carboxykomponenten, d. h. im Falle von all-D-Polypeptiden, mit einer beliebigen Aminosäuresequenz, verwendet werden können.

Infolge des vorstehend beschriebenen Auseinanderfallens der Spezifität der von dem Enzym katalysierten enzymatischen Reaktionen, d. h. Ligation und Hydrolyse, durch Vorhandensein der Abgangsgruppe an der Carboxykomponente am C-terminalen Ende ist das Enzym nach wie vor proteolytisch inaktiv gegenüber all-D-Polypeptiden, so dass hier proteolytische Nebenreaktionen unterbleiben, die ansonsten zu einer Verringerung der Ausbeute des Syntheseverfahrens führen würden. Ein weiterer Vorteil der erfindungsgemäßen Verfahren gründet sich auf der Regiospezifität der verwendeten Enzyme und des nichtvorhandenen Racemisierungsrisikos gegenüber den meisten chemischen Verfahren. Dies ist insoweit vorteilhaft, als dass Edukte mit chiralen Zentren und anderen acylierbaren Funktionen ohne experimentell aufwendige und zu zusätzlichen Nebenreaktionen führenden temporären und selektiven Blockierungsmaßnahmen eingesetzt werden können. Ausnahmen sind lediglich die in einigen Fällen notwendige Einführung N-terminaler Schutzgruppen in die Carboxykomponente, die insbesondere dann erforderlich wird, wenn die N-terminale Sequenz der Carboxykomponente eine höhere Spezifität zum Enzym aufweist als die N-terminale Sequenz der Aminokomponente. Darüber hinaus existieren im Gegensatz zu den selektiven chemischen Methoden praktisch keine Beschränkungen hinsichtlich der Sequenz der in Reaktion tretenden Edukte.

Die Begriffe Aminokomponente und Carboxykomponente, wie sie hierin verwendet werden, werden relativ zu dem zu synthetisierenden all-D-Polypeptid definiert. Dabei bezeichnet der Begriff der Aminokomponente eine chemische Verbindung, die eine Aminogruppe zur Verfügung stellt, die mit einer Carboxygruppe oder einem Derivat davon, bspw. einer Carboxygruppe, die mit einer Abgangsgruppe derivatisiert ist, einer weiteren Verbindung, d.h. der Carboxykomponente, unter Ausbildung einer Peptidbindung reagiert. Bei der Aminokomponente handelt es sich bevorzugter Weise um eine D-Aminosäure, bevorzugtererweise um ein D-Polypeptid. Im letzteren Falle weist das Polypeptid sowohl ein Amino-Ende als auch ein Carboxy-Ende auf. Das Carboxy-Ende liegt dabei entweder ungeschützt oder geschützt vor. Zur Vermeidung einer Reaktion mit einer anderen reaktiven Gruppe, wie beispielsweise der Aminogruppe eines weiteren Moleküls der Aminokomponente, liegt die Carboxylgruppe der Aminokomponente typischerweise in nichtaktivierter Form vor. Bei der Carboxykomponente handelt es sich bevorzugter Weise um eine D-Aminosäure oder, bevorzugterer Weise, um ein D-Polypeptid. Dabei ist die Carboxylfunktion oder -gruppe der Carboxykomponente, die mit der Aminogruppe, in der Regel der N-terminalen Aminogruppe, der Aminokomponente unter Ausbildung einer Peptidbindung reagiert, typischerweise aktiviert. Durch die Verknüpfung der Aminokomponente und der Carboxykomponente wird ein all-D-Polypeptid gebildet, wobei das C-terminale Ende der Aminokomponente dem C-terminalen Ende des all-D-Polypeptids entspricht und das Amino-terminale Ende der Carboxykomponente dem Amino-Ende des unter dem Einfluss des Enzyms ligierten all-D-Polypeptids entspricht.

Der Begriff all-D-Polypeptid beschreibt ein Polypeptid, welches ausschließlich aus D-Aminosäuren zusammengesetzt ist. Als D-Aminosäuren werden dabei sowohl die D-Enantiomere der natürlichen oder proteinogenen L-Aminosäuren wie auch nicht-natürliche bzw. nicht proteinogene D-Aminosäuren verwendet. Als nicht-natürliche bzw. nichtproteinogene D-Aminosäuren sind hier beispielsweise genannt:
1) D-4-Hydroxyprolin
2) 3,4-Dehydro-D-Prolin
3) D-Thiazolidin-4-Carbonsäure
4) D-Ornithin
5) D-Citrullin
6) D-Norleucin
7) D-Homoserin (und alle anderen Homo-Analoga)
8) D-δ-Hydroxylysin
9) D-Phenylglycin
10) D-Pyroglutaminsäure

Infolge der chemischen Struktur von Glycin weist dieses kein asymmetrisches Kohlenstoffatom auf, so daß eine Unterscheidung in D- und L-Glycin nicht möglich ist. Im Rahmen der vorliegenden Erfindung wird Glycin auch als D-Aminosäure betrachtet. Insoweit ist ein all-D-Polypeptid im Rahmen der vorliegenden Erfindung insbesondere auch ein solches, welches neben D-Aminosäuren ein oder mehrere Glycin-Moleküle enthält. Die Länge des all-D-Polypeptids beträgt mindestens zwei Aminosäuren. Typischerweise wird die Länge des erfindungsgemäß hergestellten all-D-Polypeptids eine Größe von 1 bis 1000, bevorzugter Weise 30 bis 1000, bevorzugterer Weise 50 bis 600 und am bevorzugtesten 100 bis 300 aufweisen.

Es ist auch im Rahmen der vorliegenden Erfindung, dass bei dem erfindungsgemäßen Verfahren als Aminokomponente und/oder Carboxykomponente PNA, peptide nucleic acid, wie beispielsweise beschrieben in Ray A et al., the FASEB Journal Vol. 14, Juni 2000, Seiten 1041-1060, beschrieben. Insoweit ist PNA ein all-D-Polypeptid und das einzelne Monomer von PNA eine D-Aminosäure im Sinne der vorliegenden Erfindung. Die verschiedenen Ausführungsformen der hierin beschriebenen Aspekte der Erfindung gelten auch für PNA und insbesondere für die Synthese von PNA. PNA ist eine Substanzklasse, die Strukturelemente von Nukleinsäuren und Peptiden bzw. Proteinen miteinander vereint. Ebenso wie bei Nukleinsäuren enthält PNA Basen (Adenin, Guanin, Uracil, Thymin, Cytosin, Inosin oder andere Pyrimidin- und Purinmodifikationen), die aber nicht mit einem Phosphodiesterrückgrat wie bei Ribonukleinsäuren oder Desoxyribonukleinsäuren, sondern mit einem Rückrat aus 2-Aminoethyl-Glycin verknüpft sind. Während die Basen der PNA in der Lage sind, mit antiparallelen Strängen von DNA und RNA zu hybridisieren, zeigt das 2-Aminoethyl-Glycin-Rückgrat peptidische Eigenschaften. So wird für die Synthese von PNAs auch die Standardchemie der Peptidsynthese eingesetzt. Auch für die PNA-Synthese gilt, dass mit zunehmender Kettenlänge die Ausbeuten an Endprodukt abnehmen, so dass das erfindungsgemäße Verfahren auch auf diese Substanzklasse mit Erfolg angewandt werden kann. Dabei ist es auch im Rahmen der Erfindung, dass die Aminokomponente PNA oder ein PNA aufbauendes Monomer ist und die Carboxykomponente eine Abgangsgruppe umfasst und aus mindestens einer D-Aminosäure, bevorzugter Weise einem all-D-Polypeptid besteht, die Aminokomponente aus mindestens einer D-Aminosäure, bevorzugter Weise einem all-D-Polypeptid besteht und die Carboxykomponente eine Abgangsgruppe umfasst und aus wenigstens einem Monomer von PNA, bevorzugter Weise einem PNA-Polymer, hierin auch als PNA-Strang bezeichnet, besteht.

Die Größe der Aminokomponente kann sowenig wie eine (D)-Aminosäure betragen. Eine obere Grenze der Länge der Aminokomponente ist nicht notwendigerweise gegeben, wird jedoch letzten Endes, wenn überhaupt, durch die Spezifität des verwendeten Enzyms sowie reaktionskinetischen Betrachtungen, wie beispielsweise Diffusionsgeschwindigkeit der Aminokomponente, bestimmt werden. Typische Größen der Aminokomponente sind dabei 1 bis 60 Aminosäuren, bevorzugter Weise 10 bis 50 Aminosäuren und bevorzugtererweise 30 bis 40 Aminosäuren. Gleiches gilt grundsätzlich für die Carboxykomponente. Es ist jedoch auch im Rahmen der vorliegenden Erfindung, dass die Länge der Aminokomponente bzw. Carboxykomponente deutlich größer ist, insbesondere bei solchen Ausführungsformen des erfindungsgemäßen Verfahrens, bei denen eine sequenzielle Protease-katalysierte all-D-Peptidfragmentligation am polymeren Träger erfolgt. Die Länge beträgt dann bevorzugter Weise ein Vielfaches der vorstehend genannten Längenbereiche, ausgedrückt als n, wobei n eine ganze Zahl bevorzugter Weise von 1 bis 20 ist. [AKB: Hier mein Vorschlag. Vernünftig?] Dabei ist es im Rahmen der vorliegenden Erfindung, dass die Aminokomponente größer, gleich oder kleiner als die Carboxykomponente ausgebildet ist,
wobei als Kriterium hierzu in der Regel die Anzahl der die Aminokomponente bzw. die Carboxykomponente ausbildenden (D)-Aminosäuren herangezogen wird. Bevorzugter Weise werden die Aminokomponente sowie die Carboxykomponente in etwa die gleiche Länge von Aminosäuren aufweisen.

Bei den erfindungsgemäßen Verfahren ist bevorzugter Weise vorgesehen, dass als Aminokomponente N-terminal ungeschützte Peptide eingesetzt werden. Bei der Carboxygruppe liegt die Abgangsgruppe typischerweise als Ester oder Amid vor, bevorzugter Weise als Ester oder Amid an der C-terminalen Carboxylgruppe der Carboxykomponente. Wie bereits ausgeführt, wird durch die spezifische Erkennung der Abgangsgruppe letztendlich die enzymatische Aktivität des Enzyms dergestalt abgeändert, dass auch all-D-Peptidfragmente anstelle der L-Fragmente durch die Protease verknüpft werden. Für Argininspezifische Proteasen, wie z.B. Clostripain, wurde für die 4-Guanidinophenylester-Abgangsgruppe eine derartige spezifitätsvermittelnde Wirkung festgestellt. Eine analoge Funktion ist auch für Amidinophenylester zu beobachten. Darüber hinaus besitzen aufgrund der Strukturhomolgie die 4-Guanidinophenylthioester- und 4-Amidinophenylthioester-Analoga eine ähnliche Wirkung und weisen zudem Vorteile bei der chemischen Synthese auf. Strukturhomologe dieser Verbindungen kommen ebenfalls als spezifitätsvermittelnde Abgangsgruppen in Betracht. Als strukturhomologe Verbindungen gelten solche mit einer basischen Gruppierung, wie beispielsweise Amino-, Amidino-, Guanidino- und Iminogruppierungen, die mit spezifitätsbestimmenden Aminosäureresten der Protease wechselwirken, d.h. insbesondere solchen Aminosäureresten, die direkt oder indirekt mit dem Substrat in Wechselwirkung treten bzw. solchen, die die katalytische Reaktion beeinflussen, und einem aliphatischen oder aromatischen Grundkörper mit z. B. einer Kettenlänge zwischen einer und sechs Methyleneinheiten bzw. Benzol-, Naphthalin- oder Indol-Grundkörpern zwischen der spezifischen basischen Gruppierung und der Ester- bzw. Amidfunktion als verknüpfendes Element zwischen der Carboxykomponente und der Abgangsgruppe. Die gesamte weitere Verfahrensweise, d.h. die Wahl der Edukte, des Puffers, des Mediums, der Reaktionszeit bzw. -Temperatur etc., ist verhältnismäßig unkritisch und kann vom Fachmann für biokatalytische Transformationen ermittelt werden.

Die Reaktion erfolgt bevorzugter Weise in wässrigen Medien oder in Lösung oder Suspension, die ggf. Anteile organischer Lösungsmittel und/oder Puffersubstanzen enthält.

Die Synthese der Carboxykomponenten mit der bevorzugter Weise Enzym-spezifischen Abgangsgruppe kann in Lösung durch Kondensation des Peptides oder der jeweiligen C-terminalen Aminosäure mit der jeweiligen Abgangsgruppe (oder geeigneten Vorstufen) oder aber am polymeren Träger, z.B. durch Verwendung von Sulfamylbutyrylaminomethyl-Safety-Catch-Harzen (vgl. R. Ingenito et al., *J. Am. Chem. Soc.* **1999,** 121, 11369) oder Oxim-Harzen (vgl. V. Cerovsky, F. Bordusa, *J. Peptide Res.* **2000,** 55, 325; V. Cerovsky et al., *ChemBioChem* **2000,** 2, 126) mit synchroner Ester- bzw. Amid-Generierung und Peptidabspaltung erfolgen. Als ablösendes Nukleophil kommt die entsprechende Alkohol-, Phenol-, Naphthol-, Indol-, Amid- oder Thiol-Komponente selbst, wie auch bereits vorgefertigte *N*^{α}-ungeschützte Aminosäureester bzw. -amide oder geeignete Vorstufen zum Einsatz. Die Synthese der als Aminokomponenten eingesetzen all-D-Peptidfragmente ist in Lösung oder durch Verwendung konventioneller Fmoc- oder Boc-Syntheseprotokolle am polymeren Träger routinemäßig möglich. Üblicherweise ist die Synthese am polymeren Träger wegen der Vorteile bei der Aufreinigung der einzelnen Zwischenprodukte einer aufwendigeren Lösungssynthese vorzuziehen.

Die erhaltenen all-D-Peptide bzw. all-D-Proteine können mit üblichen Methoden der Peptidund Proteinchemie separiert und gereinigt werden. Ggf. vorhandene Schutzgruppen können nach im Stand der Technik bekannten Verfahren entfernt werden.

Im Gegensatz zu anderen potentiell verwendbaren biokatalytischen Verfahren wird durch die erfindungsgemäße Verwendung von Enzymen und insbesondere Proteasen und Peptidasen eine hohe Syntheserate, Flexibilität, Syntheseeffizienz und Einfachheit in der Handhabung erreicht. Es ist im Rahmen der vorliegenden Erfindung, dass die bei den erfindungsgemäßen Verfahren verwendeten Aminokomponente(n) und bzw. oder Carboxykomponente(n) mit einem der erfindungsgemäßen Verfahren wiederum selbst hergestellt werden können und damit eine schrittweise Ligation des letzten Endes erwünschten all-D-Polypeptids aus verschiedenen Einzelfragmenten erreicht werden kann. Dabei ist es im Rahmen der vorliegenden Erfindung, dass auch bei dieser spezifischen Ausgestaltung des erfindungsgemäßen Verfahrens eine Immobilisierung der Aminokomponente bzw. Carboxykomponente vorgesehen ist. Bevorzugter Weise wird die Aminokomponente an eine feste Matrix immobilisiert, so dass die terminale Aminogruppe für die Reaktion mit der Carboxykomponente zur Verfügung steht. In Folge dieser Immobilisierung eines der Reaktionspartner und damit auch des Ligationsproduktes aus Aminokomponente und Carboxykomponente ergeben sich Vorteile hinsichtlich der Aufreinigung der einzelnen Zwischenprodukte, die beispielsweise durch einfaches Waschen möglich wird, wodurch die Effizienz der Synthese, insbesondere bei mehreren Ligationsschritten, erhöht wird. Bei den erfindungsgemäßen Verfahren werden, sofern üblich, die im Bereich der Synthese von Polypeptiden üblichen Schutzgruppen verwendet, wobei diese jedoch infolge der Spezifität der enzymatischen Reaktion bei einer Vielzahl von Reaktionen und Reaktionsbedingungen entfallen können. Andererseits ist es sehr wohl möglich, dass eine Entfernung der Schutzgruppen vor oder nach der Ligation der Carboxy- und/oder Aminokomponente vollständig oder teilweise erfolgt.

Die vorliegende Erfindung wird anhand der Zeichnungen und Beispiele veranschaulicht, aus denen sich weitere Merkmale, Ausführungsformen und Vorteile der Erfindung ergeben. Dabei zeigt
- Fig. 1: ein kinetisches Schema einer Protease-katalysierten Synthese von all-D-Polypeptiden,
- Fig. 2: ein Reaktionsschema zur Synthese eines all-D-Polypeptids umfassend 15 D-Aminosäuren ausgehend von einer sechs D-Aminosäuren umfassenden Carboxykomponente und einer neun D-Aminosäuren umfassenden Aminokomponente ;
- Fig.3: ein MALDI-Spektrum des unter Anwendung des erfindungsgemäßen Verfahrens und wie in Beispiel 5 beschrieben hergestellten Peptids;
- Fig. 4: eine schematische Darstellung der sequentiellen Protease-katalysierten all-D-Peptidfragmentligation am polymeren Träger;
- Fig. 5A: ein Verfahren zur Einführung einer Enzym-spezifischen Abgangsgruppe synchron zur Peptidspaltung vom polymeren Träger; und
- Fig. 5B: ein Verfahren zur Einführung einer Enzym-spezifischen Abgangsgruppe in Lösung durch Verwendung eines vollgeschützten Peptids als Vorläufer-Molekül.

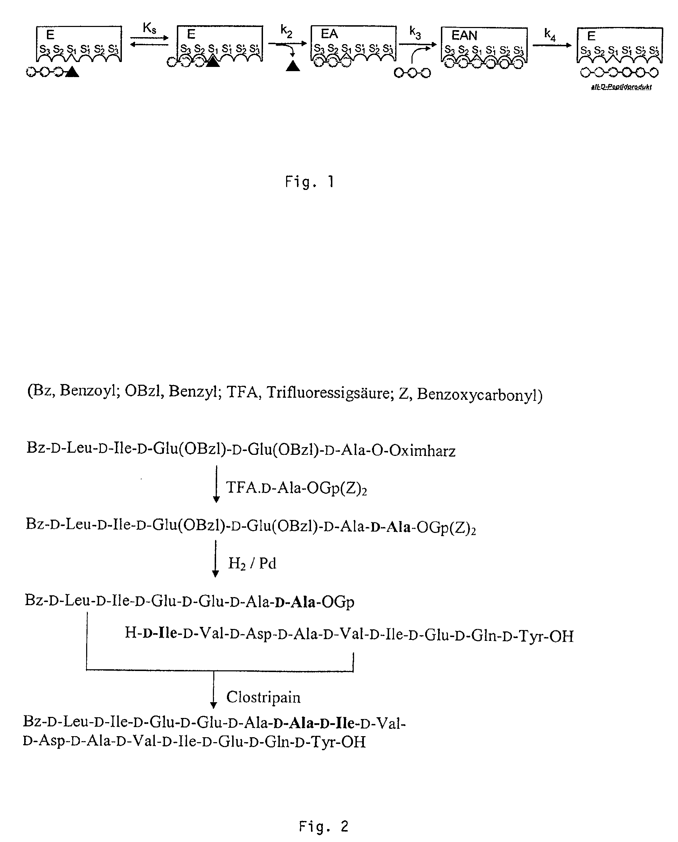

Bei dem in Fig. 1 gezeigten vereinfachten kinetischen Schema einer Protease-katalysierten Synthese von all-D-Polypeptiden durch Kondensation, hierin auch als Fragmentkondensation bezeichnet, unter Verwendung einer Carboxykomponente, welche eine Abgangsgruppe umfasst und einer Aminokomponente. Dabei zeigt (A) das Enzym E mit den Bindungsstellen S₃, S₂, S₁, S_{'1}, S_{'2} und S_{'3}. Im vorliegenden Fall besteht die Carboxykomponente aus drei individuellen D-Aminosäuren, die eine Abgangsgruppe tragen, die als Dreieck dargestellt ist. Die die Abgangsgruppe tragende Carboxykomponente bindet mit im vorliegenden Falle zwei Aminosäuren an das Enzym E (B), wobei die Abgangsgruppe spezifisch mit der Bindungsstelle S₁ am Enzym in Wechselwirkung tritt. In einem weiteren Schritt (C) kommt es zur Abspaltung der Abgangsgruppe, woraufhin sich die Carboxykomponente an der Bindungsstelle umlagert und das D-Polypeptid als Substrat akzeptiert wird. Im folgenden Schritt (D) bindet die Aminokomponente aus im vorliegenden Falle drei D-Aminosäuren an die Bindungsstellen S_{'1}, S_{'2} und S_{'3}, woraufhin eine Peptidbindung zwischen der Carboxykomponente und der Aminokomponente ausgebildet wird. In einem weiteren Schritt (E) dissoziiert das solchermaßen erhaltene all-D-Polypeptid vom Enzym ab.

Bei der in Fig. 2 dargestellten Synthese wird ausgehend von einem mit verschiedenen Schutzgruppen wie beispielsweise Benzoyl (Bz) oder Benzyl (OBzl) versehenes Pentapeptid, welches an ein Oximharz gebunden ist, mittels Trifluoressigsäure und einem mit OGp und einer Benzoxycarbonyl-Schutzgruppe versehenem D-Alanin zu einem Hexamer umgesetzt, von welchem anschließend unter Verwendung von Palladium die beiden Z-Schutzgruppen der Guanidinofunktion der Enzym-spezifischen Abgangsgruppe werden. Das solchermaßen erhaltene Hexamer weist an seinem Carboxylende die Abgangsgruppe 4-Guanidinophenylester (OGp) auf, die sodann mit dem dargestellten Nonamer als Aminokomponente unter Verwendung von Clostripain umgesetzt wird.

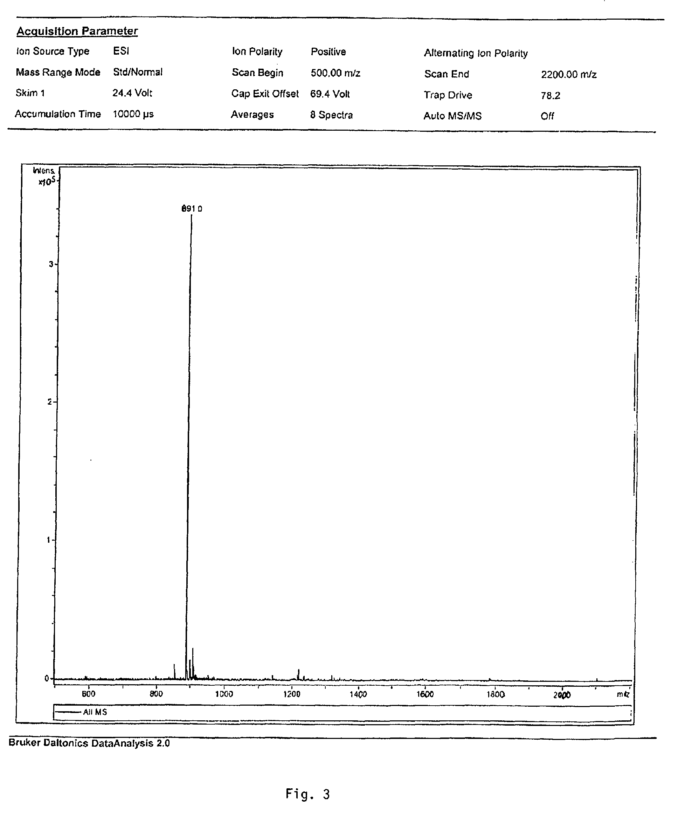

Fig. 3 zeigt das MALDI-Spektrum des gemäß dem erfindungsgemäßen Verfahren hergestellten und in Beispiel 5 beschriebenen Peptids. Die angegebene Molmasse von 891 entspricht der Hälfte des (M+2H) Signals von 1782. Die Molmasse des Peptids beträt 1780. Durch zweifache Ionisierung erscheint das Signal bei der halben Molmasse und stimmt demzufolge mit dem dargestellten Spektrum überein.

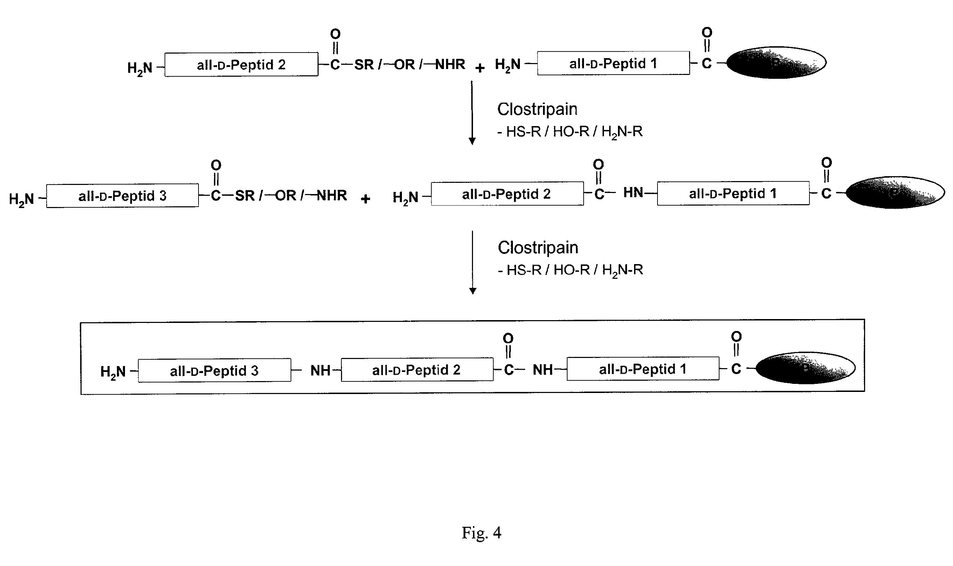

Fig. 4 zeigt ein Reaktionsschema für die sequentielle Protease-katalysierte all-D-Peptidfragmentligation an einem polymeren Träger. Dabei wird ausgehend von einem polymeren Träger, an den eine Aminokomponente, im vorliegenden Falle ein all-D-Peptid (all-D-Peptid 1), über seine Carboxygruppe an den Träger gebunden ist, mit einer Carboxykomponente umgesetzt. In dem konkret dargestellten Beispiel ist die Carboxykomponente ein all-D-Peptid, als all-D-Peptid 2 bezeichnet, welches an seiner terminalen Carboxygruppe eine Abgangsgruppe R trägt, die als Säureester, Säureamid oder Säurethioester ausgebildet ist. Unter dem Einfluss der Protease Clostripain kommt es zu einer Ligation der Amino- und Carboxykomponente und Freisetzung der Abgangsgruppe. In einem weiteren Schritt kann sodann eine weitere Carboxygruppe bereitgestellt werden, die eine gleiche oder eine verschiedene Abgangsgruppe verglichen mit derjenigen von all-D-Peptid 2 aufweisen kann. Als Aminokomponente in diesem Reaktionsansatz dient das um all-D-Peptid 2 verlängerte, an die feste Matrix gekoppelte all-D-Peptid 1. Nach Ligation mittels Clostripain wird ein im vorliegenden Fall insgesamt 3 Peptidteile oder -fragmente umfassendes all-D-Peptid erhalten, welches an die feste Matrix gebunden ist. In Folge der Immobilisierung einer der Ausgangskomponenten sowie des Ligationsproduktes ergeben sich besondere Vorteile bei der Aufreinigung der Zwischenprodukte analog zur konventionellen Festphasen-Peptidsynthese.

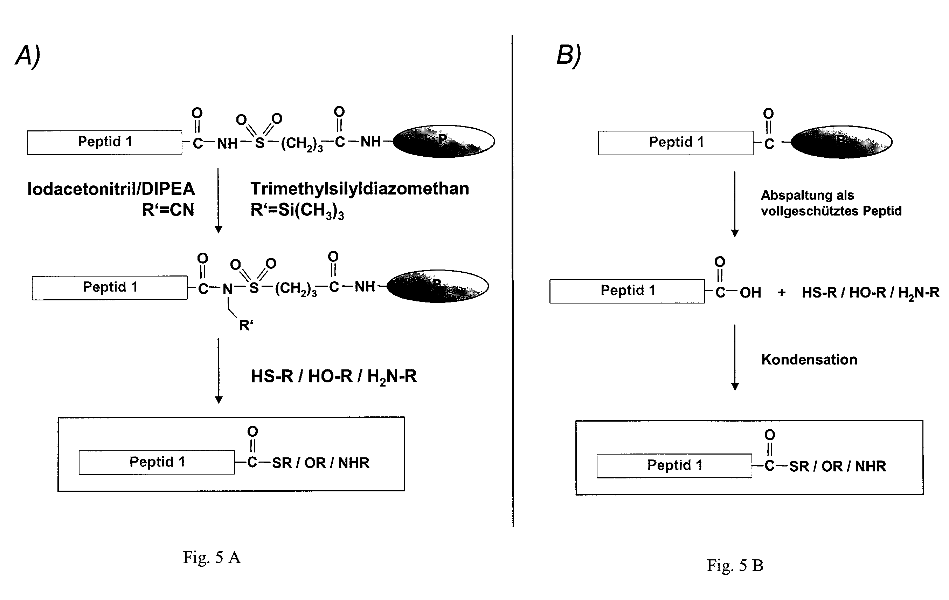

Fig. 5A zeigt eine Synthese für die Carboxykomponente, insbesondere die Einführung der Enzym-spezifischen Abgangsgruppe. Ausgehend von beispielsweise einem Sulfamylbuterylaminomethyl-Safety-Catch-Harz (kommerziell erhältlich von beispielsweise Calbiochem-Novabiochem AG) und ein daran gebundenes Peptid, hierin als Peptid 1 bezeichnet, wird entweder unter Verwendung von Jodacetonitril/DIPEA mit R'=CN oder Trimethylsilyldiazomethan mit R'=Si(CH₃)₃ ein Derivat hergestellt und nach Zusetzen einer Abgangsgruppe, beispielsweise vorliegend als Thio-, Hydroxy- oder Amin-Derivat, die Säureamidbindung zwischen dem Peptid und der festen Matrix gespalten unter gleichzeitiger Verknüpfung der Carboxygruppe von Peptid 1 mit der Abgangsgruppe, die beispielsweise sodann als Thioester, Ester oder Amid vorliegen kann.

In Fig. 5 B ist eine Alternative für die Synthese der Carboxykomponente dargestellt. Hier erfolgt die Einführung der Enzym-spezifischen Abgangsgruppe in Lösung durch Verwendung eines vollgeschützten Peptids als precursor oder Vorläufermolekül. Genauer gesagt wird nach erfolgter Festphasen-Synthese von Peptid 1 dieses von der festen Matrix abgespalten unter Ausbildung einer Carboxygruppe, die dann mit der als HS-, HO- oder H₂N-Derivat vorliegenden Abgangsgruppe kondensiert wird und so die mit einer Abgangsgruppe versehene Carboxykomponente bereitstellt, die in dem erfindungsgemäßen Verfahren verwendet werden kann.

Die vorliegende Erfindung wird im folgenden anhand von Beispielreaktionen näher ausgeführt.

### Beispiel 1 - Kondensation von Bz-D-Ala-OGp (Bz, Benzoyl; OGp, 4-Guanidinophenylester) mit ausgewählten D-Aminosäureamiden bzw. all-D-Peptiden mit Clostripain.

1 ml 0,1 M Hepes-Puffer, pH 8,0, enthaltend 0,2 M NaCl, 0,02 M CaCl₂, 5% Methanol, 2 mM Bz-D-Ala-OGp, 10 mM Aminokomponente und 15 µM Clostripain werden bei 25 °C unter pH-Kontrolle gerührt. Nach 10-60 min wird die Reaktionslösung mit 1 proz. Trifluoressigsäure in Methanol/Wasser (1:1, v/v) auf pH 2 gebracht. Die Ausbeuten werden HPLC analytisch bestimmt und sind in der nachfolgenden Tabelle 1 aufgeführt.

Die Synthese von Bz-D-Ala-OGp erfolgte analog des Syntheseprotokolles von M. Thormann et al., *Biochemistry* **1999,** 38, 6056. Die Aminokomponenten sind kommerziell erhältliche Produkte. Clostripain wurde von Amano (Japan) oder Fluka (Schweiz) bezogen.

**Tabelle 1**

| Carboxykomponente | Aminokomponente | all-D-Peptidprodukt | Ausbeute [%] |
|---|---|---|---|
| Bz-D-Ala-OGp | H-D-Phe-NH₂ | Bz-D-Ala-D-Phe-NH₂ | 90,5 |
| | H-D-Leu-NH₂ | Bz-D-Ala-D-Leu-NH₂ | 89,7 |
| | H-D-Pro-NH₂ | Bz-D-Ala-D-Pro-NH₂ | 60,7 |
| | H-(D-Leu)₂-OH | Bz-D-Ala-(D-Leu)₂-OH | 55,2 |
| | H-(D-Met)₂-OH | Bz-D-Ala-(D-Met)₂-OH | 43,1 |
| | H-(D-Ala)₃-OH | Bz-D-Ala-(D-Ala)₃-OH | 64,8 |

### Beispiel 2 - Kondensation von Bz-D-Leu-OGp (Bz, Benzoyl; OGp, 4-Guanidinophenylester) mit ausgewählten D-Aminosäureamiden bzw. all-D-Peptiden mit Clostripain.

Die Reaktionsbedingungen entsprechen denjenigen von Beispiel 1. Die verschiedenen Aminokomponenten, die mit Bz-D-Leu-OGp umgesetzt werden, die Reaktionsprodukte sowie die Ausbeuten sind in Tabelle 2 dargestellt.

**Tabelle 2**

| Carboxykomponente | Aminokomponente | all-D-Peptidprodukt | Ausbeute [%] |
|---|---|---|---|
| Bz-D-Leu-OGp | H-D-Phe-NH₂ | Bz-D-Leu-D-Phe-NH₂ | 66,3 |
| | H-D-Leu-NH₂ | Bz-D-Leu-D-Leu-NH₂ | 65,7 |
| | H-D-Pro-NH₂ | Bz-D-Leu-D-Pro-NH₂ | 46,2 |
| | H-(D-Leu)₂-OH | Bz-D-Leu-(D-Leu)₂-OH | 41,9 |
| | H-(D-Met)₂-OH | Bz-D-Leu-(D-Met)₂-OH | 37,3 |
| | H-(D-Ala)₃-OH | Bz-D-Leu-(D-Ala)₃-OH | 45,8 |

### Beispiel 3 - Kondensation von Bz-D-Phe-OGp (Bz, Benzoyl; OGp, 4-Guanidinophenylester) mit ausgewählten D-Aminosäureamiden bzw. all-D-Peptiden mit Clostripain.

Die Reaktionsbedingungen entsprechen denjenigen von Beispiel 1. Die verschiedenen Aminokomponenten, die mit Bz-D-Phe-OGp umgesetzt werden, die Reaktionsprodukte sowie die Ausbeuten sind in Tabelle 3 dargestellt.

**Tabelle 3**

| Carboxykomponente | Aminokomponente | all-D-Peptidprodukt | Ausbeute [%] |
|---|---|---|---|
| Bz-D-Phe-OGp | H-D-Phe-NH₂ | Bz-D-Phe-D-Phe-NH₂ | 67,4 |
| | H-D-Leu-NH₂ | Bz-D-Phe-D-Leu-NH₂ | 61,9 |
| | H-D-Pro-NH₂ | Bz-D-Phe-D-Pro-NH₂ | 44,0 |
| | H-(D-Leu)₂-OH | Bz-D-Phe-(D-Leu)₂-OH | 41,6 |
| | H-(D-Met)₂-OH | Bz-D-Phe-(D-Met)₂-OH | 38,1 |
| | H-(D-Ala)₃-OH | Bz-D-Phe-(D-Ala)₃-OH | 46,6 |

### Beispiel 4 - Kondensation von Bz-Gly-OGp (Bz, Benzoyl; OGp, 4-Guanidinophenylester) mit ausgewählten D-Aminosäureamiden bzw. all-D-Peptiden mit Clostripain.

Die Reaktionsbedingungen entsprechen denjenigen von Beispiel 1. Die verschiedenen Aminokomponenten, die mit Bz-D-Gly-OGp umgesetzt werden, die Reaktionsprodukte sowie die Ausbeuten sind in Tabelle 4 dargestellt.

**Tabelle 4**

| Carboxykomponente | Aminokomponente | all-D-Peptidprodukt | Ausbeute [%] |
|---|---|---|---|
| Bz-Gly-OGp | H-D-Phe-NH₂ | Bz-Gly-D-Phe-NH₂ | 98,4 |
| | H-D-Leu-NH₂ | Bz-Gly-D-Leu-NH₂ | 99,1 |
| | H-D-Pro-NH₂ | Bz-Gly-D-Pro-NH2 | 72,6 |
| | H-(D-Leu)₂-OH | Bz-Gly-(D-Leu)₂-OH | 45,8 |
| | H-(D-Met)₂-OH | Bz-Gly-(D-Met)₂-OH | 43,6 |
| | H-(D-Ala)₃-OH | Bz-Gly-(D-Ala)₃-OH | 62,4 |

### Beispiel 5 - Kondensation von Bz-D-Leu-D-Ile-D-Glu-D-Glu-D-Ala-D-Ala-OGp (Bz, Benzoyl; OGp, 4-Guanidinophenylester) mit H-D-Ile-D-Val-D-Asp-D-Ala-D-Val-D-Ile-D-Glu-D-Gln-D-Tyr-OH mit Clostripain.

Die diesem Beispiel zugrundeliegende Reaktion ist in Fig. 1 dargestellt.

0,32 ml des in DMSO (Dimethylsulfoxid) gelösten Hexapeptidesters Bz-D-Leu-D-Ile-D-Glu-D-Glu-D-Ala-D-Ala-OGp (0.01 mmol) werden zu der Aminokomponente H-D-Ile-D-Val-D-Asp-D-Ala-D-Val-D-Ile-D-Glu-D-Gln-D-Tyr-OH, gelöst in 0,465 ml 0,2 M Veronal-Puffer (pH 8,5) gegeben und auf 25 °C temperiert. Durch Zugabe von Clostripain (15 µM im Reaktionsansatz) wird die Reaktion gestartet und für weitere 1,5 Stunden bei 25 °C gerührt. Durch Zugabe einer 2 proz. Trifluoressigsäure in Methanol/Wasser (1:1, v/v) Lösung wird der pH-Wert auf 2 verringert und die Reaktion dadurch beendet. Die Ausbeute wurden HPLCchromatographisch bestimmt und betrug 65,5%. Die Identität des Syntheseproduktes wurde mittels MALDI-MS verifiziert. Das Ergebnis ist in Fig. 3 dargestellt.

Die in der vorstehenden Beschreibung, den Ansprüchen sowie den Zeichnungen offenbarten Merkmale der Erfindung können sowohl einzeln als auch in beliebigen Kombinationen für die Verwirklichung der Erfindung in ihren verschiedenen Ausführungsformen wesentlich sein.

## Patentansprüche

1. Verfahren zur Synthese eines all-D-Polypeptids unter Verwendung eines Enzyms umfassend die Schritte:
(a) Bereitstellen einer Aminokomponente, wobei die Aminokomponente aus mindestens einer D-Aminosäure, bevorzugter Weise einem all-D-Polypeptid besteht,
(b) Bereitstellen der Carboxykomponente, wobei die Carboxykomponente eine Abgangsgruppe umfasst und aus mindestens einer D-Aminosäure, bevorzugter Weise einem all-D-Polypeptid besteht, und
(c) Umsetzen der Aminokomponente und der Carboxykomponente mit dem Enzym unter Ausbildung einer Peptidbindung zwischen der Aminokomponente und der Carboxykomponente und unter Abspaltung der Abgangsgruppe von der Carboxykomponente, wobei das all-D-Polypeptid erhalten wird.

2. Verfahren nach Anspruch 1, weiter umfassend als Schritt:
(d) Isolieren des all-D-Polypeptids.

3. Verfahren zur Synthese von PNA unter Verwendung eines Enzyms umfassend die Schritte:
(a) Bereitstellen einer Aminokomponente, wobei die Aminokomponente aus mindestens einem PNA-Monomer, bevorzugter Weise einem ersten PNA-Strang besteht,
(b) Bereitstellen einer Carboxykomponente, wobei die Carboxykomponente eine Abgangsgruppe umfasst und die Carboxykomponente aus mindestens einem PNA-Monomer, bevorzugter Weise einem zweiten PNA-Strang besteht, und
(c) Umsetzen der Aminokomponente und der Carboxykomponente mit dem Enzym unter Ausbildung einer Peptidbindung zwischen der Aminokomponente und der Carboxykomponente unter Abspaltung der Abgangsgruppe von der Carboxykomponente, wobei ein ligierter PNA-Strang erhalten wird.

4. Verfahren nach Anspruch 3, weiter umfassend als Schritt:
(d) Isolieren des ligierten PNA-Stranges.

5. Verfahren nach einem der Ansprüche 1 bis 4, **dadurch gekennzeichnet, dass** das Enzym ausgewählt ist aus der Gruppe, die Proteasen, Peptidasen und Hydrolasen umfasst.

6. Verfahren nach einem der Ansprüche 1 bis 5, **dadurch gekennzeichnet, dass** das Enzym ausgewählt ist aus der Gruppe, die Cysteinproteasen und Serinproteasen umfasst.

7. Verfahren nach einem der Ansprüche 1 bis 6, **dadurch gekennzeichnet, dass** das Enzym Clostripain, insbesondere Clostripain aus *Clostridium histolyticum* ist.

8. Verfahren nach einem der Ansprüche 1 bis 6, **dadurch gekennzeichnet, dass** das Enzym eine Prolylendopeptidase, insbesondere eine Prolylendopeptidase aus *Flavobacterium meningoseptum* ist.

9. Verfahren nach einem der Ansprüche 1 bis 8, **dadurch gekennzeichnet, dass** die Abgangsgruppe ausgewählt ist aus der Gruppe, die 4-Guanidinophenylester , 4-Amidinophenylester, 4-Guanidinophenylthioester und 4-Amidinophenylthioester und Strukturhomologe davon umfasst.

10. Verfahren nach einem der Ansprüche 1 bis 9, **dadurch gekennzeichnet, dass** die die Abgangsgruppe enthaltende Carboxykomponente die folgende Struktur aufweist:
Y-(D-Xaa)ₙ-R
wobei Y = eine N-terminale Schutzgruppe, insbesondere Benzoyl ist,
Xaa = eine beliebige α-Aminosäure, β-Aminosäure oder ein Derivat derselben ist
R = eine Abgangsgruppe ist, insbesondere eine Abgangsgruppe ist, die ausgewählt ist aus der Gruppe, die 4-Guanidinophenylester, 4-Amidinophenylester, 4-Guanidinophenylthioester, 4-Amidinophenylthioester und Strukturhomologe davon umfasst
n = eine ganze Zahl von 1 bis 100

11. Verfahren nach einem der Ansprüche 1 bis 10, **dadurch gekennzeichnet, dass** die N^{α}-Aminofunktion der Aminokomponente ungeschützt vorliegt, wobei N^{α}-Aminofunktion mit der Carboxyfunktion der Carboxykomponente reagiert.

12. Verfahren zur Synthese eines all-D-Polypeptids unter Verwendung eines Enzyms umfassend die Schritte
(a) Bereitstellen einer Aminokomponente, wobei die Aminokomponente aus mindestens einer D-Aminosäure, bevorzugter Weise einem all-D-Polypeptid besteht,
(b) Bereitstellen der Carboxykomponente, wobei die Carboxykomponente eine Abgangsgruppe umfasst und die Carboxykomponente aus mindestens einer D-Aminosäure, bevorzugter Weise aus einem all-D-Polypeptid besteht, und
(c) Umsetzen der Aminokomponente und der Carboxykomponente mit dem Enzym unter Ausbildung einer Peptidbindung zwischen der Aminokomponente und der Carboxykomponente und Abspalten der Abgangsgruppe von der Carboxykomponente, wobei das all-D-Polypeptid erhalten wird,
wobei die Aminokomponente hergestellt ist nach einem Verfahren gemäß einem der Ansprüche 1 bis 11.

13. Verfahren zur Synthese von PNA unter Verwendung eines Enzyms umfassend die Schritte
(a) Bereitstellen einer Aminokomponente, wobei die Aminokomponente aus mindestens einem PNA-Monomer, bevorzugter Weise einem ersten PNA-Strang besteht,
(b) Bereitstellen einer Carboxykomponente, wobei die Carboxykomponente eine Abgangsgruppe umfasst und die Carboxykomponente aus mindestens einem PNA-Monomer, bevorzugter Weise aus einem zweiten PNA-Strang besteht, und
(c) Umsetzen der Aminokomponente und der Carboxykomponente mit dem Enzym unter Ausbildung einer Peptidbindung zwischen der Aminokomponente und der Carboxykomponente und Abspalten der Abgangsgruppe von der Carboxykomponente,
wobei die Aminokomponente hergestellt ist nach einem Verfahren gemäß einem der Ansprüche 1 bis 11.

14. Verfahren zur Synthese eines all-D-Polypeptids unter Verwendung eines Enzyms, insbesondere nach Anspruch 12, umfassend die Schritte
(a) Bereitstellen einer Aminokomponente, wobei die Aminokomponente aus mindestens einer D-Aminosäure, bevorzugter Weise einem all-D-Polypeptid besteht,
(b) Bereitstellen der Carboxykomponente, wobei die Carboxykomponente eine Abgangsgruppe umfasst und die Carboxykomponente aus mindestens einer D-Aminosäure, bevorzugter Weise aus einem all-D-Polypeptid besteht, und
(c) Umsetzen der Aminokomponente und der Carboxykomponente mit dem Enzym unter Ausbildung einer Peptidbindung zwischen der Aminokomponente und der Carboxykomponente und Abspalten der Abgangsgruppe von der Carboxykomponente, wobei das all-D-Polypeptid erhalten wird,
wobei die Carboxykomponente hergestellt ist nach einem Verfahren gemäß einem der Ansprüche 1 bis 11.

15. Verfahren zur Synthese von PNA unter Verwendung eines Enzyms umfassend die Schritte
(a) Bereitstellen einer Aminokomponente, wobei die Aminokomponente aus mindestens einem PNA-Monomer, bevorzugter Weise einem ersten PNA-Strang besteht,
(b) Bereitstellen einer Carboxykomponente, wobei die Carboxykomponente eine Abgangsgruppe umfasst und die Carboxykomponente aus mindestens einem PNA-Monomer, bevorzugter Weise aus einem zweiten PNA-Strang besteht, und
(c) Umsetzen der Aminokomponente und der Carboxykomponente mit dem Enzym unter Ausbildung einer Peptidbindung zwischen der Aminokomponente und der Carboxykomponente und Abspalten der Abgangsgruppe von der Carboxykomponente,
wobei die Carboxykomponente hergestellt ist nach einem Verfahren gemäß einem der Ansprüche 1 bis 11.

16. Verwendung eines Enzyms zur Synthese eines all-D-Polypeptids und/oder von PNA

17. Verwendung nach Anspruch 16, wobei das all-D-Polypeptid und/oder die PNA durch Ligation einer Aminokomponente und einer Carboxykomponente hergestellt wird und die Carboxykomponente eine Abgangsgruppe umfasst.

18. Verwendung nach Anspruch 12 oder 17, **dadurch gekennzeichnet, dass** die Abgangsgruppe ausgewählt ist aus der Gruppe, die 4- Guanidinophenylester, 4-Amidinophenylester, 4-Guanidinophenylthioester und 4-Amidinophenylthioester und Strukturhomologe davon umfasst.

19. Verwendung einer Abgangsgruppe, die ausgewählt ist aus der Gruppe, die 4-Guanidinophenylester, 4-Amidinophenylester, 4-Guanidinophenylthioester und 4-Amidinophenylthioester und Strukturhomologe davon umfasst, zur Synthese eines all-D-Polypeptids und/oder von PNA unter Verwendung eines Enzyms.

20. Verwendung nach Anspruch 19 **dadurch gekennzeichnet, dass** die Abgangsgruppe an einem all-D-Polypeptid vorhanden ist und das die Abgangsgruppe tragende all-D-Polypeptid als Carboxykomponente mit einer D-Aminosäure oder mit einem all-D-Polypeptid als Aminokomponente unter Verwendung eines Enzyms umgesetzt wird.

21. Verwendung nach Anspruch 19, **dadurch gekennzeichnet, dass** die Abgangsgruppe an einem PNA-Strang vorhanden ist und der die Abgangsgruppe tragende PNA-Strang als Carboxykomponente mit einem PNA-Monomer oder mit einem zweiten PNA-Strang als Aminokomponente unter Verwendung eines Enzyms umgesetzt wird.

22. Verwendung nach einem der Ansprüche 16 bis 20, **dadurch gekennzeichnet, dass** das Enzym Clostripain, insbesondere Clostripain aus *Clostridium histolyticum* ist.

23. Verwendung nach einem der Ansprüche 16 bis 20, **dadurch gekennzeichnet, dass** das Enzym Prolylendopeptidase, insbesondere Prolylendopeptidase aus *Flavobacterium meningoseptum* ist.
